# EUROPEAN PATENT SPECIFICATION

(11) **EP 0 451 008 B1**
(45) Date of publication and mention of the grant of the patent: **28.06.1995**
(21) Application number: 91400770.3
(22) Date of filing: 21.03.1991
(51) Int. Cl.: C07C 311/35, A61K 31/18

(54) **8-Sulfamylmethylene-2-amino tetralins**
8-Sulfamylmethylen-2-aminotetraline
8-Sulfamylméthylène-2-aminotétralines

(30) Priority: 21.03.1990 EP 90400781
(43) Date of publication of application: 09.10.1991
(73) Proprietor: MERRELL DOW PHARMACEUTICALS INC., Cincinnati Ohio 45215-6300 (US)
(72) Inventor: Hibert, Marcel, F-67114 Eschau (FR); Petty, Margaret A., F-67000 Strasbourg (FR); Jones, C. Richard, F-67370 Truchtersheim (FR)
(74) Representative: Gillard, Marie-Louise

(56) References cited:
- WO-A-84/04247
- DE-A- 3 320 521

## Description

This invention relates to novel 8-substituted-2-amino tetralins, to the methods for their preparation and to their use in the treatment of migraine.

More specifically, this invention relates to compounds of the formula 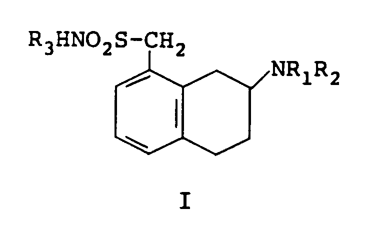
the enantiomers and mixtures thereof and the pharmaceutically acceptable salts thereof wherein
R₁ and R₂ each represents hydrogen or C₁₋₇ lower alkyl, and
R₃ is C₁₋₇ lower alkyl.

The term lower alkyl includes the straight, branched-chain and cyclized alkyl moieties having up to 7 carbon atoms including cyclohexylmethyl, preferably propyl, methyl, ethyl and butyl. Pharmaceutically acceptable salts include all those salts normally utilized for pharmaceutical preparations, particularly those salts derived from organic or inorganic acids, for example hydrochlorides, hydrobromides, sulphates, nitrates, phosphates, formates, mesylates, citrates, benzoates, fumarates, maleates, and salts with, e.g., p-toluene sulphonic acid.

The separation of the optical isomers can be achieved by such techniques as fractional distillation, chromatographic methods and preferably by enantio selective crystallization using optically pure acids such as (+) or (-) binaphthyl phosphonic acid. Typically the amine and optically pure binaphthyl phosphonic acid dissolved in acetone or warm isopropanol are mixed and maintained between 4 and 25°C until crystallization of one of the two diastereoisomeric salts. The solid is separated and recrystallized and the optically active free amine is recovered by extraction with ether from an aqueous solution of potassium carbonate. The other enantiomer is recovered from the mother liquors, basified and recrystallized with the other enantiomer of the binaphthyl phosphonic acid.

In their end-use application it is preferred to utilize the compounds in their enantiomeric pure state rather than mixtures thereof, although the mixtures are still useful but at increased dosages depending upon the proportionate mixture.

In general the compounds of this invention may be prepared by standard procedures analogously known in the art. In practice the compounds may conveniently be prepared by the following reaction scheme. 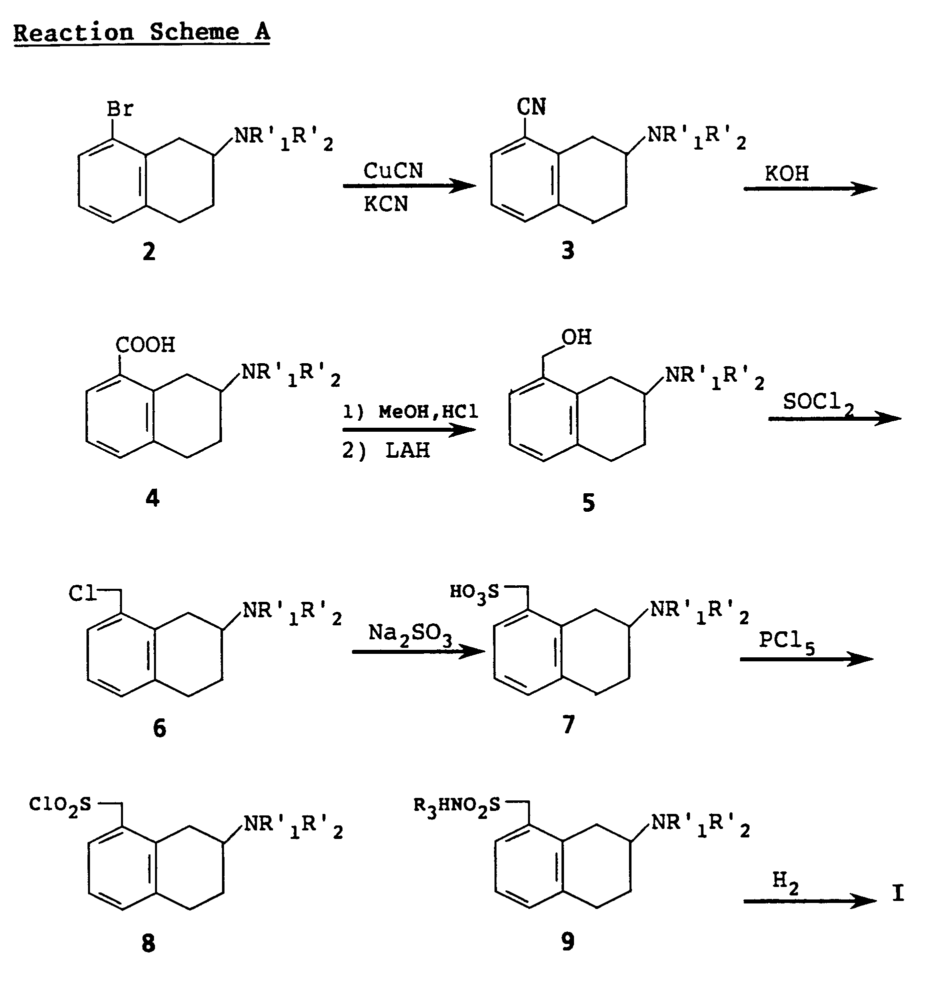
wherein R₁, R₂ and R₃ are as previously defined and R′₁ and R′₂ are C₁₋₇ alkyl or a removable amino protecting group, preferably benzyl, or together form a phthalamido protecting group (removed by treatment with hydrazine).

The synthesis is initiated by the reaction of 8-bromo-2-R'₁R'₂ aminotetralins with copper cyanide to obtain the corresponding 8-cyano aminotetralins, said reaction being effected by standard nucleophilic replacement reactions well known in the art. The resulting 8-cyano compounds (3) are hydrolized to their corresponding acids (4) by reaction with a strong base such as potassium hydroxide and the resulting acids are esterified and reduced to the corresponding alcohols (5) using lithium aluminum hydride (LAH). These alcohols, upon treatment with thionyl chloride are converted to their methylchloride (6) which upon treatment with sodium sulfate yields the corresponding sulfonic acid (7) which upon treatment with phosphorous pentachloride, gives the sulfonyl chloride (8). The sulfonyl chloride is treated with the appropriate amine of formula NH₂R₃ to produce the sulphonamide (9). In the event there are any N-protecting groups (e.g., benzyl) on the 2-position amine then such groups are readily removed by standard hydrogenation techniques to yield the desired compounds of Formula I. In those instances wherein it is desired to prepare compounds wherein R₁ and R₂ are both a C₁₋₇ alkyl, e.g., di-n-propyl amino, it is preferred to alkylate the free amine (following removal of the R'₁, R'₂-protecting groups) using a C₁₋₇ alkyl halide, preferably an alkyl iodide in a basic medium. Of course, it is also obvious that in those instances wherein it is desired to prepare a 2-position dialkyl amino (i.e., R₁ and R₂ are a C₁₋₇ alkyl) then it may also be convenient to start with compounds of formula (2) wherein R'₁ and R'₂ represent a C₁₋₇ alkyl. In those instances wherein it is desired to prepare compounds wherein one of R₁ and R₂ is H, then it is preferred to utilize the process of the foregoing reaction scheme wherein R'₁ is a protecting group and R'₂ is the desired C₁₋₇ alkyl moiety. The foregoing reactions are standard reactions very familar to those of ordinary skill in the art, the reaction conditions of which being specifically illustrated by the specific examples herein detailed.

Alternatively, the compounds may be synthesized by the procedures depicted in the following reaction scheme. 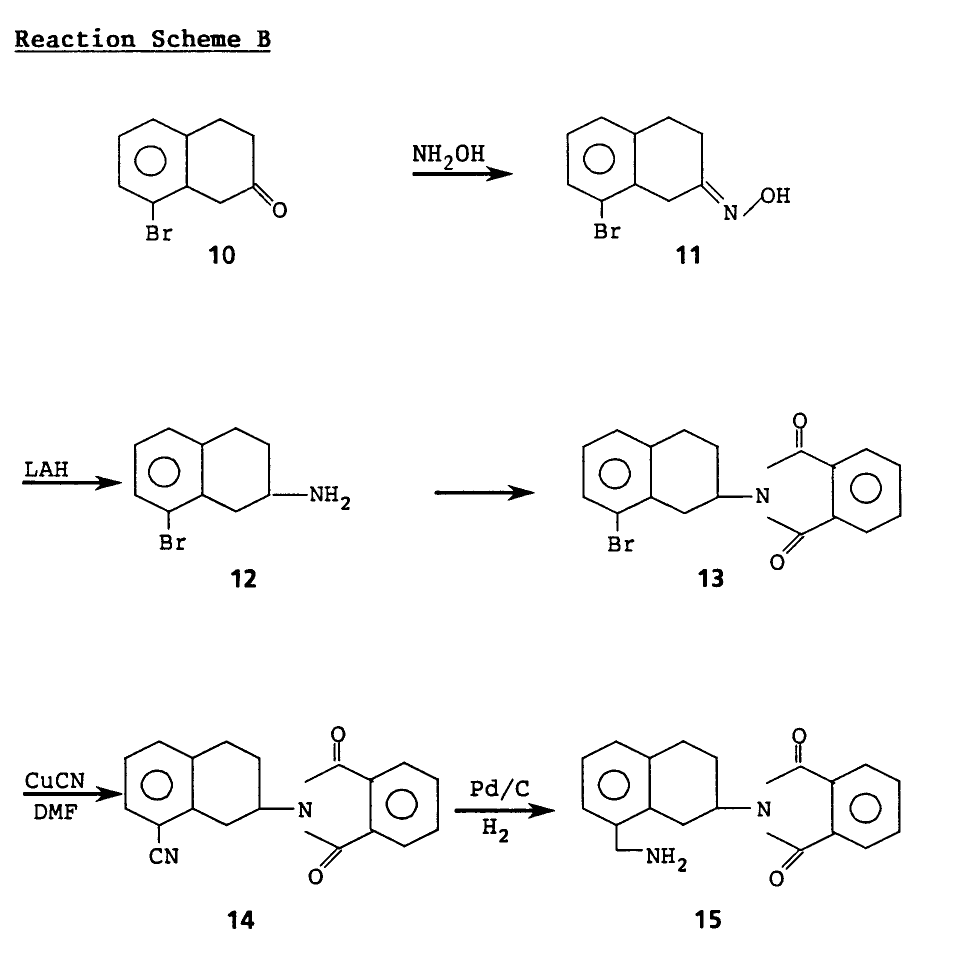 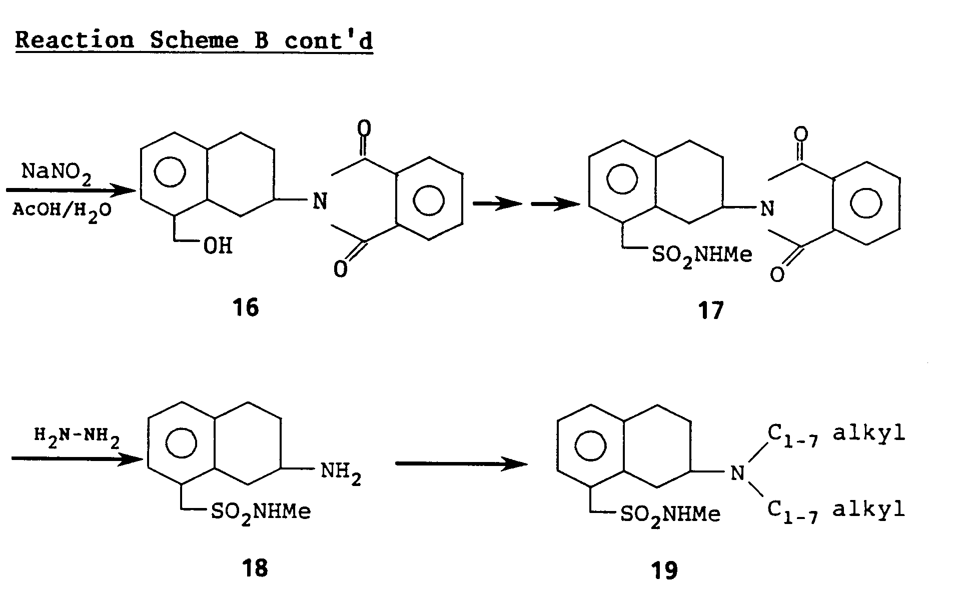
In essence, Reaction Scheme B represents a series of reactions analogously known in the art wherein compound (10) is converted to its oxime, the oxime reduced with lithium aluminum hydride (LAH), the amine protected by its conversion to its phthalamido derivative (13), the bromo replaced with a cyano radical by reaction with cuprous cyanide in dimethyl formamide, the cyano moiety of (14) is converted to its amine by catalytic hydrogenation (Pd/C), the resulting aminomethyl radical is converted to its hydroxymethyl by reaction with sodium nitrite in acetic acid and water, and as in reaction Scheme A, the hydroxymethyl moiety is converted to its methylsulfonylmethyl derivative (17) which upon treatment with hydrazine, the phthalamido protecting group is removed to yield the desired product (18). In practice it is also advantageous to utilize a trifluoroacetamide as a protecting group which may be removed by standard procedures. The resulting product (18) may then be converted to its dialkylamino derivative (e.g. its di-n-propyl derivative) by reaction with an appropriate alkyl halide (e.g., 1-iodo propane) in a basic medium at slightly elevated temperatures.

The following examples detail the foregoing reaction schemes.

### Example 1

### 2-Dimethylamino-8-cyano-1,2,3,4-tetrahydronaphthalene

Under nitrogen, 2-dimethylamino-8-amino-1,2,3,4-tetrahydronaphthalene (24 mmol) is dissolved in 33% sulfuric acid (120 mmol) and cooled down to 0°C. Sodium nitrite (24 mmol) in water (5 mmol) is carefully added under stirring at 0°C. After 30 minutes at this temperature, the mixture is slowly poured into a solution of cuprous cyanide (32 mmol) and sodium cyanide (177 mmol) in water (40 ml) warmed at 50°C.
The mixture is stirred at 50°C for one hour under a nitrogen atmosphere and then poured into 500 ml iced water. The pH of the solution is brought to 10 with 2N sodium hydroxide. Extraction with dichloromethane, washing of the organic phase with water, drying over sodium sulfate and evaporation to dryness affords the crude expected product. Purification by flash chromatography on silica gel (using toluene/methanol as eluent) affords the pure expected product.

### Example 2

### 2-Dimethylamino-1,2,3,4-tetrahydronaphthalene-8-carboxylic acid

2-Dimethylamino-8-cyano-1,2,3,4-tetrahydronaphthalene (1 mmol), potassium hydroxide (2 mmol) and water (2 mmol) in ethylene glycol (1 ml) are warmed at 150°C for 20 hours under a nitrogen atmosphere. After cooling, water is added and the aqueous phase is extracted with dichloromethane, acidified to pH 1-2 with 1N hydrochloride and extracted again with dichloromethane. Ammonia (25%) is then used to bring the pH of theaqueous phase to 5. Water is then evaporated under vacuum and the solid residue is taken up in dry tetrahydrofuran. The unsoluble salts are filtered and tetrahydrofuran evaporated to dryness, yielding the expected acid as an oil.

### Example 3

### 2-Dimethylamino-8-methoxycarbonyl-1,2,3,4-tetrahydronaphthalene

2-Dimethylamino-1,2,3,4-tetrahydronaphthyl-8-carboxylic acid (1.1 mmol) is dissolved in 30 ml of dry methanol. A saturated solution of hydrochloride in ether (3 ml) is added and the mixture is stirred at room temperature for 24 hours. The solvents are evaporated under vacuum and the residue is dissolved in dichloromethane. Successive washing with 0.1N sodium hydroxide followed by drying over sodium sulfate and evaporation leads to the expected product as a colorless oil.

### Example 4

### 2-Dimethylamino-8-hydroxymethyl-1,2,3,4-tetrahydronaphthalene

Lithium aluminum hydride (0.4 mmol) is suspended under argon atmosphere in dry ether. A solution of 2-dimethylamino-8-methoxycarbonyl-1,2,3,4-tetrahydronaphthalene (0.7 mmol) in ether (1 ml) is added, the mixture is stirred at room temperature for 5 hours. A saturated solution of sodium sulfate in water is then added. After stirring for 30 minutes the mixture is extracted with ethyl acetate and dichloromethane. After drying over sodium sulfate, the organic phase is evaporated to dryness. Purification by flash chromatography on silica gel (methylene chloride/methanol) affords the pure expected product as an oil.

### Example 5

### 2-Dimethylamino-8-chloromethyl-1,2,3,4-tetrahydronaphthalene

2-Dimethylamino-8-hydroxymethyl-1,2,3,4-tetrahydronaphthalene hydrochloride (1 mmol) is dissolved in thionyl chloride (5 ml) at O°C and the mixture is stirred overnight at room temperature. The mixture is evaporated to dryness and the residue is dissolved with ether and water. The aqueous phase is washed with ether, basified with potassium bicarbonate and extracted with ethyl acetate. The organic extract is then washed with water, dried over sodium sulfate and evaporated to dryness, to yield the expected chloride which is used without further purification.

### Example 6

### 2-Dimethylamino-8-sulfinomethyl-1,2,3,4-tetrahydronaphthalene

2-Dimethylamino-8-chloromethyl-1,2,3,4-tetrahydronaphthalene hydrochloride (3.0 mmol) and sodium sulfite (3.3 mmol) in water (15 ml) are warmed under reflux for 2 hours. After cooling and concentration the product precipitates, is filtered, washed with acetone and ether, dried under vacuum and used as such for the next step.

### Example 7

### 2-Dimethylamino-8-chlorosulfonylmethyl-1,2,3,4-tetrahydronaphthalene

2-Dimethylamino-8-sulfinomethyl-1,2,3,4-tetrahydronaphthalene hydrochloride (2.75 mmol) and phosphorous pentaiodide(3.2 mmol) are mixed at 0°C. The addition is exothermic. The mixture is progressively warmed and maintained at 80°C for 1.5 hours. Most of phosphoryl chloride is eliminated by evaporation under vacuum (16 mmHg). The mixture is then cooled in an ice bath and water is carefully added to the oily residue. The mixture is then evaporated to dryness under vacuum and used as such for the next step.

### Example 8

### -2-dimethylamino-8-methylaminosulfonylmethyl-1,2,3,4-tetrahydronaphthalene

2-Dimethylamino-8-chlorosulfinomethyl-1,2,3,4-tetrahydronaphthalene (approximately 2.7 mmol) in dry tetrahydrofuran is added to an excess of methylamine in dry tetrahydrofuran under stirring at -20°C. A solid precipitates, the mixture is stirred at room temperature for 12 hours. After evaporation to dryness, the crude solid is dissolved in water and methylene chloride. The organic phase is washed with 5% ammonium hydroxide and brine, dried over sodium sulfate and concentrated to dryness. The crude oil which is obtained is purified by flash chromatography on silica gel (eluent: gradient of methanol in methylene chloride). The hydrochloride of the pure free base is prepared in hydrochloride/isopropanol and recrystallized from methanol/ethyl acetate/ether.

### Example 9

### 8-Bromo-2-tetralone, oxime

8-Bromo-2-tetralone (10 mmol) is dissolved in chloroform. Oxime hydrochloride (12 mmol) in water is added. The mixture is vigorously stirred for 12 hours. The organic phase is recovered, washed with 10% sodium bicarbonate and brine and evaporated to dryness to afford the expected product.

### Example 10

### 2-Amino-8-bromo-1,2,3,4-tetrahydronaphthalene

Lithium aluminum hydride (3 mmol) is suspended in ether under an argon atmosphere. The oxime of 8-bromo-2-tetralone (10 mmol) in ether is added dropwise under stirring at 0°C. After 5 hours at room temperature, a minimum amount of a saturated solution of sodium sulfate in water is added. Dry sodium sulfate is added and the solid residue is extracted with ethyl acetate. The amine is extracted with 10% hydrochloride, basified with 10% sodium hydroxide and extracted with dichloromethane. Drying over sodium sulfate and evaporation leads to the pure expected amine.

### Example 11

### 2-Phthalimido-8-bromo-1,2,3,4-tetrahydronaphthalene

2-Amino-8-bromo-1,2,3,4-tetrahydronaphthalene (10 mmol) is dissolved in dichloromethane (50 ml) with triethylamine (25 mmol) at 0°C. Dichlorophthalyl chloride (10 mmol) in dichloromethane (30 ml) is slowly added under rapid stirring. The mixture is stirred at 0°C for 30 minutes and at room temperature for 3 hours. Ether, in excess, is added, the precipitated solid is filtered. The organic phase is washed with 5% sodium hydroxide, 5% hydrochloride and brine, dried over sodium sulfate and evaporated to dryness to afford the expected compound.

### Example 12

### 2-Phthalimido-8-cyano-1,2,3,4-tetrahydronaphthalene

2-Phthalimido-8-bromo-1,2,3,4-tetrahydronaphthalene (1 mmol) and cuprous cyanide (1.2 mmol) are refluxed in dimethylformamide for 6 hours. The resulting hot brown mixture is poured into a solution of hydrated ferric chloride and concentrated hydrochloride in water. The mixture is stirred at room temperature for 30 minutes, to decompose the complex. An excess of water is added and the mixture is extracted with toluene and ether. The organic phase is washed with water, 10% potassium carbonate and brine, dried over sodium sulfate and evaporated to dryness. The crude product is purified by flash chromatography (ethyl acetate/dichloromethane as eluent).

### Example 13

### 2-Phthalimido-8-aminomethyl-1,2,3,4-tetrahydronaphthalene

2-Phthalimido-8-cyano-1,2,3,4-tetrahydronaphthalene (8 mmol) is dissolved in ethanol. Methyl bisulfite (8 mmol) and 10% Pd over charcoal (50 mg) are added. Hydrogenation at atmospheric pressure yields the title amine as the methanesulfonate salt.

### Example 14

### 2-Phthalimido-8-hydroxymethyl-1,2,3,4-tetrahydronaphthalene

2-Phthalimido-8-aminomethyl-1,2,3,4-tetrahydronaphthalene (8 mmol) is dissolved in diluted acetic acid. Sodium nitrite (8.5 mmol) in water is added. The reaction is stirred at room temperature for 3 hours and extracted with ether. The organic phase is washed with 5% hydrochloride, 5% sodium hydroxide and brine, dried over sodium sulfate and evaporated to dryness. The expected alcohol is purified by flash chromatography (ethyl acetate/dichloromethane as eluent) and is obtained pure as a colorless oil.

### Example 15

### 2-Phthalimido-8-methylaminosulfonylmethyl-1,2,3,4-tetrahydronaphthalene

Following essentially the same procedure as for Examples 5 to 8 but substituting 2-phthalimido-8-hydroxymethyl-1,2,3,4-tetrahydronaphthalene for 2-dimethylamino-8-hydroxymethyl-1,2,3,4-tetrahydronaphthalene, the title compound is prepared.

### Example 16

### 2-Amino-8-methylaminosulfonylmethyl-1,2,3,4-tetrahydronaphthalene

2-Phthalimido-8-methylaminosulfonylmethyl-1,2,3,4-tetrahydronaphthalene is dissolved in ethyl acetate. Hydrazine hydrate (1.05 equivalent) is added and the mixture is refluxed for 3 hours. The solvent is evaporated and the crude solid is dissolved in methanol (20 ml). Concentrated hydrochloride (1 ml) is added and the mixture is stirred at room temperature for 2 hours. After evaporation to dryness, the solid residue is dissolved in water and ethyl acetate. The aqueous phase is washed with ethyl acetate, basified with 5% sodium hydroxide and extracted with dichloromethane. The organic phase washed with water and brine, dried over sodium sulfate and evaporated to dryness will afford the expected amine as an oil.Purification by flash chromatography (methanol/ethyl acetate as eluent) and recrystallization of the hydrochloride salt from isopropanol/ethyl acetate/ether will afford the pure title compound.

### Example 17

### 2-(N,N-Methyl-benzylamino)-8-methylaminosulfonylmethyl-1,2,3,4-tetrahydronaphthalene

Following essentially the procedure of Examples 1 to 8 but substituting 2-[N,N-methyl-benzylamino]-8-methylaminosulfonylmethyl-1,2,3,4-tetrahydronaphthalene (10 mmol) for 2-dimethylamino-8-bromo-1,2,3,4-tetrahydronaphthalene, the title compound is prepared.

### Example 18

### 2-Methylamino-8-methylaminosulfonylmethyl-1,2,3,4-tetrahydronaphthalene

2-(N,N-methyl-benzylamino)-8-methylaminosulfonylmethyl-1,2,3,4-tetrahydronaphthalene (10 mmol) is dissolved in ethanol with methane sulfonic acid (10 mmol). The mixture is hydrogenated under atmospheric pressure to afford the title compound as a methane sulfonic salt.

### Example 19

### 2-(N′,N′-Dipropylamino)-8-methylaminosulfonylmethyl-1,2,3,4-tetrahydronaphthalene

The primary amine of Example 16 (120 mg, 0.47 mmol) is suspended in acetonitrile (7 ml) under an argon atmosphere. Triethylamine (0.43 ml, 3 mmol) and 1-iodo-propane (0.21 ml, 2.1 mmol) are added, and the mixture is warmed under reflux overnight. The resulting mixture is concentrated to dryness, extracted with methylene chloride, washed with 5% sodium carbonate, dried over sodium sulfate and evaporated to dryness. The resulting crude compound is purified by flash chromatography on silica gel using MeOH/CH₂Cl₂ : 5/95 as eluent. The pure amine is obtained as a yellow oil (120 mg, 75%) which crystallizes. The oxalate salt is crystallized from Et₂O/i-PrOH.
m.p. 149°C.

In general the compounds of this invention represent a very select sub-group of compounds which are generically embraced within the scope of European Patent Application 0 272534-A2. However, EPO Application 0 272534 does not specifically teach any compound within the scope of this application nor does it specifically illustrate how the compounds of this application can be prepared.

In general the compounds of this invention are potent and selective 5HT₁-like agonists useful in treating and/or preventing pain putatively associated with abnormal cranial vascular flow changes, particularly in migraine and related disorders such as for example, cluster headache.

Using standard *in vitro* and *in vivo* laborating essay methodology for testing compounds for their potential use in the treatment of migraine as well as by comparison with compounds known to have a beneficial effect in patients suffering from migraine and related disorders, it is determined that the compounds of this invention will be useful at doses in the range of about 0.25 to 100 mg per kilogram of body weight per day, preferably 0.5 to 20 mg per kilogram of body weight per day. The compounds may be administered in divided daily doses, generally 3 to 4 times per day. Of course, it is to be appreciated that it may be necessary to make routine variations to the dosages depending on the age and weight of the patient, as well as the severity of the condition to be treated. Although calculations of doses are naturally predicated on the weight of the base, it is preferred that the compounds be administered in the form of a pharmaceutically acceptable salt, preferably those salts formed with hydrochloric, hydrobromic, succinic and methanesulfonic acid.

The compounds of the invention may be formulated for parenteral administration by injection, preferably intravenous or subcutaneous injection , e.g., by bolus injection or continuous intravenous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents and/or agents to adjust the tonicity of the solution. Alternatively, the active ingredient may be in powder form for constitution with a suitable vehicle, e.g., sterile pyrogen-free water, before use.

The compounds of the invention may also be formulated in rectal compositions such as suppositories or retention enemas, e.g., containing conventional suppository bases such as coco butter or other glycerides.

Tablets for sub-lingual administration may be formulated in a similar manner to those for oral administration.

For intra-nasal administration the compounds of the invention may be used as a liquid spray or in the form of drops.

Dosages of the compounds of the invention for rectal, sublingual or intranasal administration to man (of average body weight e.g., about 70 kg) for the treatment of migraine may be similar to those described previously for oral administration.

For administration by inhalation the compounds according to the invention are conveniently delivered in the form of an aerosol spray presentation from pressurised packs, with the useof a suitable propellant, e.g., dichlorodifluoromethane, trichlorofluoromethane, dichlorotetrafluoroethane, carbon dioxide or other suitable gas, or from a nebuliser. In the case of a pressurised aerosol the dosage unit may be determined by providing a valve to deliver a metered amount. Capsules and cartridges of, for example, gelatin for use in an inhaler or insuflator may be formulated containing a powder mix of a compound of the invention and a suitable powder base such as lactose or starch.

Aerosol formulations are preferably arranged as that each metered dose or "puff" delivered from a pressurized aerosol contains 0.2 mg to 2 mg of a compound of the invention, and each dose administered via capsules and cartridges in an insufflator or an inhaler contains 0.2 mg to 20 mg of a compound of the invention. Administration may be several times daily, for example from 2 to 8 times, giving, for example, 1, 2 or 3 doses, each time. The overall daily dose by inhalation will be similar to that for oral administration.

The compounds of the invention may, if desired, be administered in combination with one or more other therapeutic agents, such as analgesics, anti-inflammatory agents and antinauseants.

Additionally, the compounds of this invention may be utilized for the treatment of hypertension, depression and mania using doses based upon standard *in vitro* and *in vivo* assay methodology, said doses being within the same range as outlined above.

Accordingly, the invention also provides a pharmaceutical composition adapted for use in medicine which comprises the compound of formula (I) and/or a physiologically acceptable salt or solvate (e.g., hydrate) thereof, formulated for administration by any convenient route. Such compositions may be formulated in conventional manner using one or more pharmaceutically acceptable carriers or excipients. The compounds according to the invention may be formulated for oral, sublingual, parenteral, rectal or intra-nasal administration, or in a form suitable for administration are preferred.

The pharmaceutical compositions for oral administration may take the form of, for example, tablets or capsules prepared by conventional means with pharmaceutically acceptable excipients such as bindings agents (e.g., pregelatinised maize starch, polyvinylpyrrolidone or hydroxypropyl methylcellulose) fillers (e.g., lactose, sucrose, mannitol, corn starch, microcrystalline cellulose or calcium hydrogen phosphate) lubricants (e.g. stearic acid, polyethylene glycol, magnesium stereate, talc or silica), disintegrants (e.g., potato starch, sodium starch glycollate or croscarmellose sodium), or wetting agents (e.g., sodium lauryl sulphate). The tablets may be coated by methods well known in the art. Liquid preparations for oral administration may take the form of, for example, aqueous or oily solutions, syrups, elixirs, emulations or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives, glucose/sugar syrup, gelatin, aluminium stereate gel, or hydrogenated edible fats), emulsifying agents (e.g., lecithin acacia or sorbitan mono-oleate), non-aqueous vehicles (e.g., almond oil, oily esters, ethyl) alcohol or fractionated vegetable oils), and preservatives (e.g., methyl or propyl p-hydroxybenzoates or sorbic acid). The liquid preparations may also contain conventional buffers flavoring, coloring and sweetening agents as appropriate.

As is true for any class of compounds suitable for use as pharmaceutically effective agents, certain members are preferred over others. In this instance it is preferred that R₃ is methyl or ethyl while it is preferred that both R₁ and R₂ are both n-propyl or methyl, or R₁ is hydrogen and R₂ is methyl, n-propyl, ethyl or butyl.

## Claims (Claims for the following Contracting State(s): AT, BE, CH, LI, DE, DK, FR, GB, IT, LU, NL, SE)

1. A compound of the formula 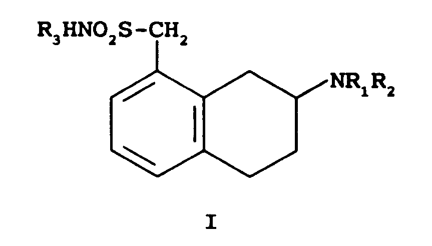 the enantiomers and mixtures thereof and the pharmaceutically acceptable salts thereof wherein
R₁ and R₂ each represents hydrogen or C₁₋₇ lower alkyl, and
R₃ is C₁₋₇ lower alkyl.

2. A compound of claim 1 wherein R₁ and R₂ are C₁₋₇ alkyl.

3. A compound of claim 1 wherein R₁ and R₂ are H.

4. A compound of claim 1 wherein R₁ is H and R₂ is C₁₋₇ alkyl.

5. A compound of claim 1 wherein R₁ and R₂ are n-propyl.

6. A compound of claim 5 wherein R₃ is methyl.

7. A compound of claim 1 wherein R₁, R₂ and R₃ are methyl.

8. A compound of claim 1 wherein R₁ is H and R₂ is methyl.

9. A compound of formula I according to claim 1, which is the 2-(N',N'-Dipropylamino)-8-methylaminosulfonylmethyl-1,2,3,4-tetrahydronaphthalene.

10. A method for preparing a compound of the formula 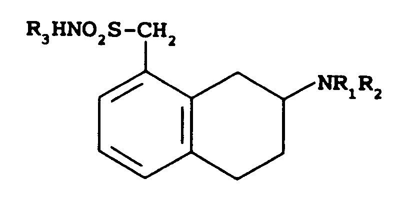 the enantiomers and mixtures thereof and the pharmaceutically acceptable salts thereof wherein
R₁ and R₂ each represents hydrogen or C₁₋₇ lower alkyl, and
R₃ is C₁₋₇ lower alkyl,
which comprises
(a) reacting a compound of the formula 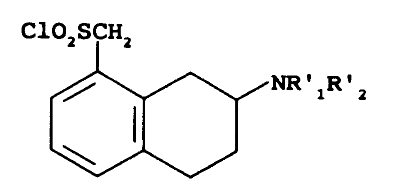 with an amine of the formula NH₂R₃,
wherein R'₁ and R'₂ are C₁₋₇ alkyl or a removable amino protecting group, followed by the removal of any protecting group,
(b) optionally N-alkylating a compound of the formula 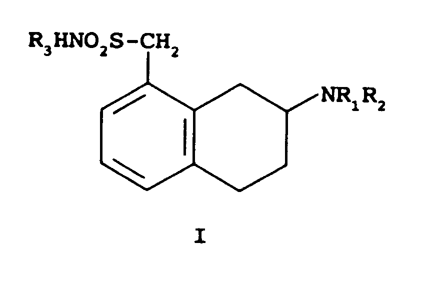 wherein R₁ and R₂ are both hydrogen
with a C₁₋₇ alkyl halide,
(c) optionally converting the resulting compound (I) into its enantiomers or pharmaceutically acceptable salts.

11. A method for preparing a compound of formula 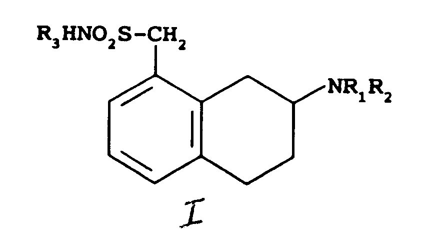 the enantiomers and mixtures thereof and the pharmaceutically acceptable salts thereof wherein
R₁ and R₂ each represents hydrogen or C₁₋₇ lower alkyl, and
R₃ is a methyl group,
which comprises :
(a) reacting a compound of formula (18) : 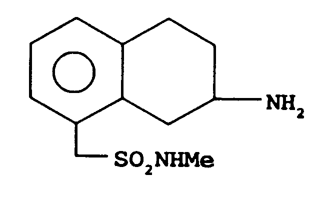 with an appropriate alkyl halide in a basic medium at slightly elevated temperatures,
(b) optionally converting the resulting compound (I) into its enantiomers or pharmaceutically acceptable salts.

12. The use of the compounds of claims 1 to 9 as pharmaceutically active agent.

13. The use of the compounds of claims 1 to 9 for the preparation of medicines useful for the treatment of migraine.

## Claims (Claims for the following Contracting State(s): ES)

1. A method for preparing a compound of the formula 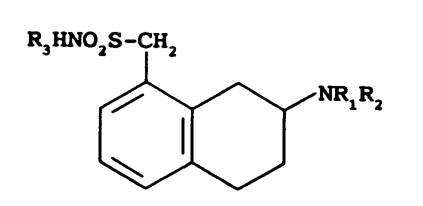 the enantiomers and mixtures thereof and the pharmaceutically acceptable salts thereof wherein
R₁ and R₂ each represents hydrogen or C₁₋₇ lower alkyl, and
R₃ is C₁₋₇ lower alkyl,
which comprises
(a) reacting a compound of the formula 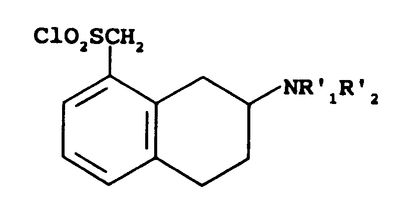 with an amine of the formula NH₂R₃,
wherein R'₁ and R'₂ are C₁₋₇ alkyl or a removable amino protecting group, followed by the removal of any protecting group,
(b) optionally N-alkylating a compound of the formula 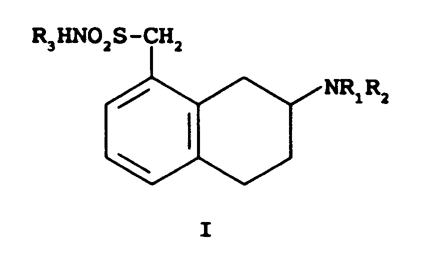 with a C₁₋₇ alkyl halide
wherein R₁ and R₂ are both hydrogen,
(c) optionally converting the resulting compound (I) into its enantiomers or pharmaceutically acceptable salts.

2. Process for preparing a compound of formula 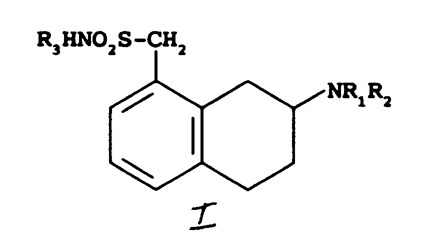 the enantiomers and mixtures thereof and the pharmaceutically acceptable salts thereof wherein
R₁ and R₂ each represents hydrogen or C₁₋₇ lower alkyl, and
R₃ is a methyl group,
which comprises :
(a) reacting a compound of formula (18) : 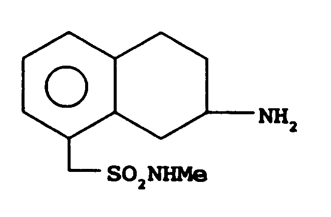 with an appropriate alkyl halide in a basic medium at slightly elevated temperatures,
(b) optionally converting the resulting compound (I) into its enantiomers or pharmaceutically acceptable salts.

3. Process according to claim 2, in which the appropriate alkyl halide is 1-iodo-propane and the resulting product is the 2-(N',N'-Dipropylamino)-8-methylaminosulfonyl methyl-1,2,3,4-tetrahydronaphthalene.

4. Use of the compounds obtained by the process according to any one of claims 1 to 3 for the preparation of medicines useful for the treatment of migraine.

## Claims (Claims for the following Contracting State(s): GR)

1. A compound of the formula 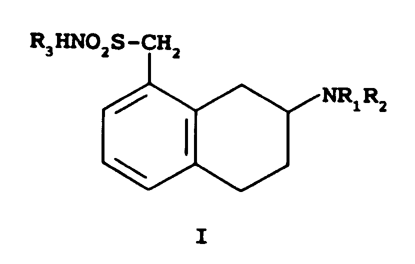 the enantiomers and mixtures thereof and the pharmaceutically acceptable salts thereof wherein
R₁ and R₂ each represents hydrogen or C₁₋₇ lower alkyl, and
R₃ is C₁₋₇ lower alkyl.

2. A compound of claim 1 wherein R₁ and R₂ are C₁₋₇ alkyl.

3. A compound of claim 1 wherein R₁ and R₂ are H.

4. A compound of claim 1 wherein R₁ is H and R₂ is C₁₋₇ alkyl.

5. A compound of claim 1 wherein R₁ and R₂ are n-propyl.

6. A compound of claim 5 wherein R₃ is methyl.

7. A compound of claim 1 wherein R₁, R₂ and R₃ are methyl.

8. A compound of claim 1 wherein R₁ is H and R₂ is methyl.

9. A compound of formula I according to claim 1, which is the 2-(N',N'-Dipropylamino)-8-methylaminosulfonylmethyl-1,2,3,4-tetrahydronaphthalene.

10. A method for preparing a compound of the formula 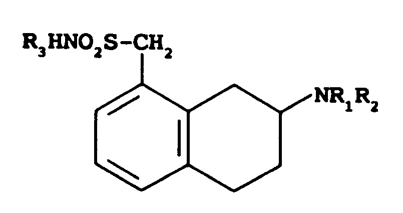 the enantiomers and mixtures thereof and the pharmaceutically acceptable salts thereof wherein
R₁ and R₂ each represents hydrogen or C₁₋₇ lower alkyl, and
R₃ is C₁₋₇ lower alkyl,
which comprises
(a) reacting a compound of the formula 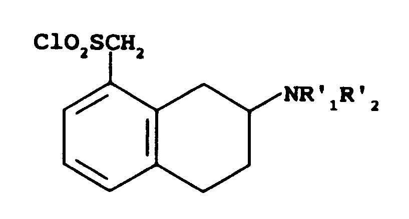 with an amine of the formula NH₂R₃,
wherein R'₁ and R'₂ are C₁₋₇ alkyl or a removable amino protecting group, followed by the removal of any protecting group,
(b) optionally N-alkylating a compound of the formula 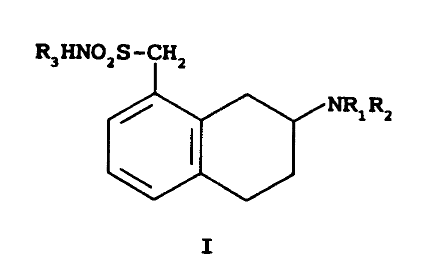 wherein R₁ and R₂ are both hydrogen
with a C₁₋₇ alkyl halide,
(c) optionally converting the resulting compound (I) into its enantiomers or pharmaceutically acceptable salts.

11. A method for preparing a compound of formula 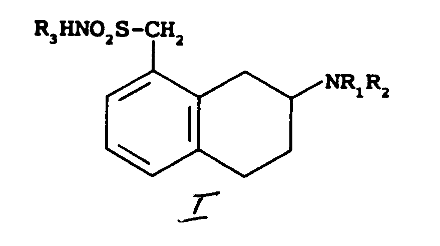 the enantiomers and mixtures thereof and the pharmaceutically acceptable salts thereof wherein
R₁ and R₂ each represents hydrogen or C₁₋₇ lower alkyl, and
R₃ is a methyl group,
which comprises :
(a) reacting a compound of formula (18) : 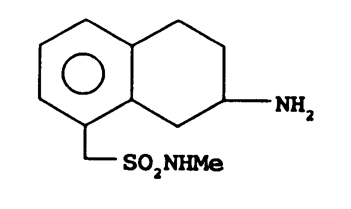 with an appropriate alkyl halide in a basic medium at slightly elevated temperatures,
(b) optionally converting the resulting compound (I) into its enantiomers or pharmaceutically acceptable salts.

12. The use of the compounds of claims 1 to 9 for the preparation of medicines useful for the treatment of migraine.

## Patentansprüche (Patentansprüche für folgende(n) Vertragsstaat(en): AT, BE, CH, LI, DE, DK, FR, GB, IT, LU, NL, SE)

1. Verbindung der Formel 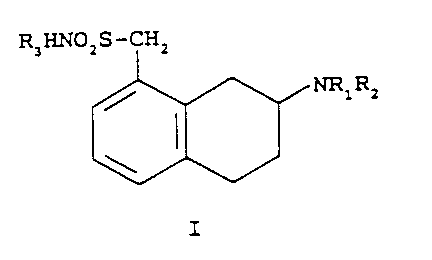 die Enantiomere und Gemische davon und die pharmazeutisch verträglichen Salze davon, wobei
R₁ und R₂ jeweils ein Wasserstoffatom oder einen C₁₋₇-Niederalkylrest wiedergeben und
R₃ einen C₁₋₇-Niederalkylrest bedeutet.

2. Verbindung nach Anspruch 1, wobei R₁ und R₂ einen C₁₋₇-Alkylrest darstellen.

3. Verbindung nach Anspruch 1, wobei R₁ und R₂ ein Wasserstoffatom bedeuten.

4. Verbindung nach Anspruch 1, wobei R₁ ein Wasserstoffatom darstellt und R₂ einen C₁₋₇-Alkylrest bedeutet.

5. Verbindung nach Anspruch 1, wobei R₁ und R₂ eine n-Propylgruppe darstellen.

6. Verbindung nach Anspruch 5, wobei R₃ eine Methylgruppe bedeutet.

7. Verbindung nach Anspruch 1, wobei R₁, R₂ und R₃ eine Methylgruppe darstellen.

8. Verbindung nach Anspruch 1, wobei R₁ ein Wasserstoffatom bedeutet und R₂ eine Methylgruppe darstellt.

9. Verbindung der Formel I nach Anspruch 1, nämlich 2-(N',N'-Dipropylamino)-8-methylaminosulfonylmethyl-1,2,3,4-tetrahydronaphthalin.

10. Verfahren zur Herstellung einer Verbindung der Formel 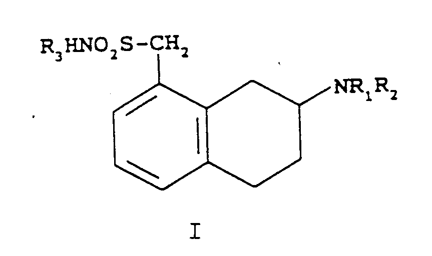 der Enantiomere und Gemische davon und der pharmazeutisch verträglichen Salze davon, wobei
R₁ und R₂ jeweils ein Wasserstoffatom oder einen C₁₋₇-Niederalkylrest wiedergeben und
R₃ einen C₁₋₇-Niederalkylrest bedeutet,
umfassend
(a) die Umsetzung einer Verbindung der Formel 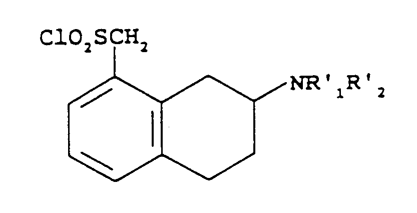 mit einem Amin der Formel NH₂R₃,
wobei R'₁ und R'₂ einen C₁₋₇-Alkylrest oder eine entfernbare Aminoschutzgruppe bedeuten, gefolgt vom Entfernen beliebiger Schutzgruppen,
(b) gegebenenfalls die N-Alkylierung einer Verbindung der Formel 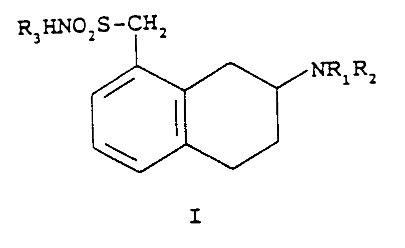 wobei R₁ und R₂ beide ein Wasserstoffatom bedeuten, mit einem C₁₋₇-Alkylhalogenid,
(c) gegebenenfalls Umwandlung der erhaltenen Verbindung (I) in ihre Enantiomere oder pharmazeutisch verträglichen Salze.

11. Verfahren zur Herstellung einer Verbindung der Formel 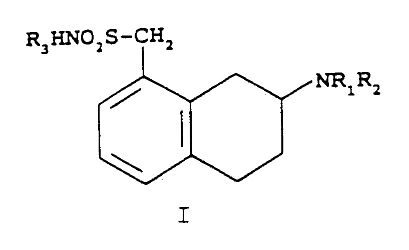 der Enantiomere und Gemische davon und der pharmazeutisch verträglichen Salze davon, wobei
R₁ und R₂ jeweils ein Wasserstoffatom oder einen C₁₋₇-Niederalkylrest wiedergeben und
R₃ eine Methylgruppe bedeutet,
umfassend:
(a) die Umsetzung einer Verbindung der Formel (18) 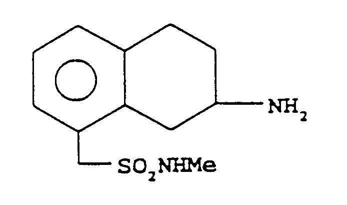 mit einem geeigneten Alkylhalogenid in einem basischen Medium bei leicht erhöhten Temperaturen,
(b) gegebenenfalls Umwandlung der erhaltenen Verbindung (I) in ihre Enantiomere oder pharmazeutisch verträglichen Salze.

12. Verwendung der Verbindungen nach den Ansprüchen 1 bis 9 als pharmazeutischer Wirkstoff.

13. Verwendung der Verbindungen nach den Ansprüchen 1 bis 9 zur Herstellung von Arzneien, die zur Behandlung von Migräne verwendbar sind.

## Patentansprüche (Patentansprüche für folgende(n) Vertragsstaat(en): ES)

1. Verfahren zur Herstellung einer Verbindung der Formel 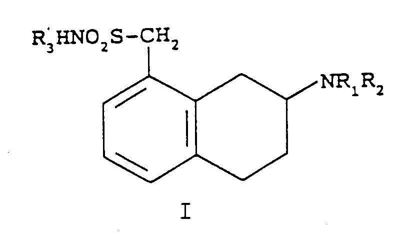 der Enantiomere und Gemische davon und der pharmazeutisch verträglichen Salze davon, wobei
R₁ und R₂ jeweils ein Wasserstoffatom oder einen C₁₋₇-Niederalkylrest darstellen und
R₃ einen C₁₋₇-Niederalkylrest bedeutet,
das umfaßt
(a) die Umsetzung einer Verbindung der Formel 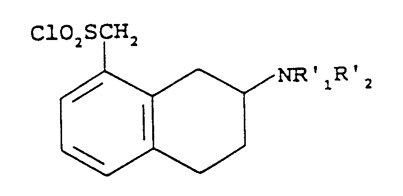 mit einem Amin der Formel NH₂R₃,
wobei R'₁ und R'₂ einen C₁₋₇-Alkylrest oder eine entfernbare Aminoschutzgruppe darstellen, gefolgt vom Entfernen beliebiger Schutzgruppen,
(b) gegebenenfalls die N-Alkylierung einer Verbindung der Formel 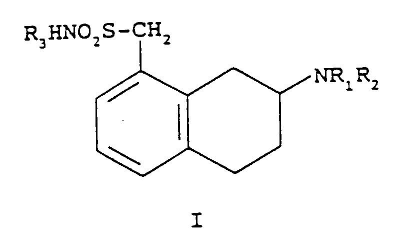 mit einem C₁₋₇-Alkylhalogenid,
wobei R₁ und R₂ beide ein Wasserstoffatom bedeuten
(c) gegebenenfalls Umwandlung der erhaltenen Verbindung (I) in ihre Enantiomere oder pharmazeutisch verträglichen Salze.

2. Verfahren zur Herstellung einer Verbindung der Formel 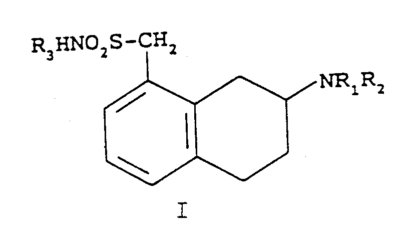 der Enantiomere und Gemische davon und der pharmazeutisch verträglichen Salze davon, wobei
R₁ und R₂ jeweils ein Wasserstoffatom oder einen C₁₋₇-Niederalkylrest darstellen und
R₃ eine Methylgruppe bedeutet,
umfassend :
(a) die Umsetzung einer Verbindung der Formel (18) 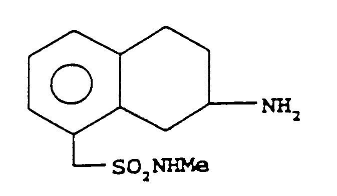 mit einem geeigneten Alkylhalogenid in einem basischen Medium bei leicht erhöhten Temperaturen,
(b) gegebenenfalls Umwandlung der erhaltenen Verbindung (I) in ihre Enantiomere oder pharmazeutisch verträglichen Salze.

3. Verfahren nach Anspruch 2, bei dem das geeignete Alkylhalogenid 1-Jodpropan ist und das erhaltene Produkt das 2-(N',N'-Dipropylamino)-8-methylaminosulfonylmethyl-1,2,3,4-tetrahydronaphthalin ist.

4. Verwendung der durch das Verfahren nach einem der Ansprüche 1 bis 3 erhaltenen Verbindungen zur Herstellung von Arzneien, die zur Behandlung von Migräne verwendbar sind.

## Patentansprüche (Patentansprüche für folgende(n) Vertragsstaat(en): GR)

1. Verbindung der Formel 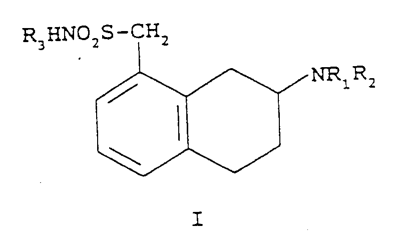 die Enantiomere und Gemische davon und die pharmazeutisch verträglichen Salze davon, wobei
R₁ und R₂ jeweils ein Wasserstoffatom oder einen C₁₋₇-Niederalkylrest darstellen und
R₃ einen C₁₋₇-Niederalkylrest bedeutet.

2. Verbindung nach Anspruch 1, wobei R₁ und R₂ einen C₁₋₇-Alkylrest darstellen.

3. Verbindung nach Anspruch 1, wobei R₁ und R₂ ein Wasserstoffatom bedeuten.

4. Verbindung nach Anspruch 1, wobei R₁ ein Wasserstoffatom darstellt und R₂ einen C₁₋₇-Alkylrest bedeutet.

5. Verbindung nach Anspruch 1, wobei R₁ und R₂ eine n-Propylgruppe darstellen.

6. Verbindung nach Anspruch 5, wobei R₃ eine Methylgruppe bedeutet.

7. Verbindung nach Anspruch 1, wobei R₁, R₂ und R₃ eine Methylgruppe darstellen.

8. Verbindung nach Anspruch 1, wobei R₁ ein Wasserstoffatom darstellt und R₂ eine Methylgruppe bedeutet.

9. Verbindung der Formel I nach Anspruch 1, nämlich das 2-(N',N'-Dipropylamino)-8-methylaminosulfonylmethyl-1,2,3,4-tetrahydronaphthalin.

10. Verfahren zur Herstellung einer Verbindung der Formel 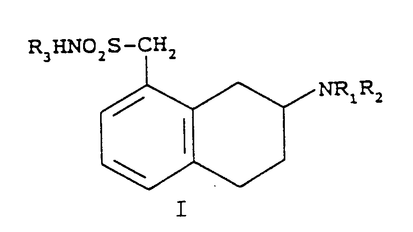 der Enantiomere und Gemische davon und der pharmazeutisch verträglichen Salze davon, wobei
R₁ und R₂ jeweils ein Wasserstoffatom oder einen C₁₋₇-Niederalkylrest darstellen und
R₃ einen C₁₋₇-Niederalkylrest bedeutet,
das umfaßt
(a) die Umsetzung einer Verbindung der Formel 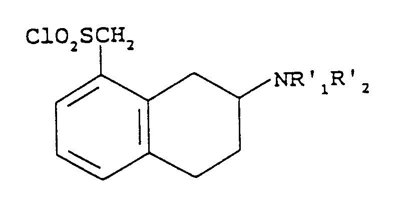 mit einem Amin der Formel NH₂R₃,
wobei R'₁ und R'₂ einen C₁₋₇-Alkylrest oder eine entfernbare Aminoschutzgruppe darstellen, gefolgt vom Entfernen beliebiger Schutzgruppen,
(b) gegebenenfalls die N-Alkylierung einer Verbindung der Formel 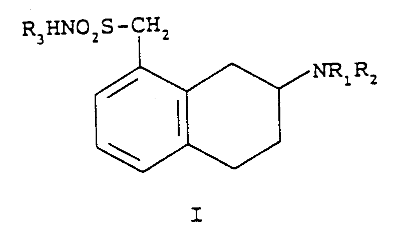 wobei R₁ und R₂ beide ein Wasserstoffatom bedeuten,
mit einem C₁₋₇-Alkylhalogenid
(c) gegebenenfalls Umwandlung der erhaltenen Verbindung (I) in ihre Enantiomere oder pharmazeutisch verträglichen Salze.

11. Verfahren zur Herstellung einer Verbindung der Formel 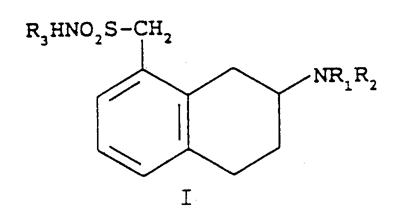 der Enantiomere und Gemische davon und der pharmazeutisch verträglichen Salze davon, wobei
R₁ und R₂ jeweils ein Wasserstoffatom oder einen C₁₋₇-Niederalkylrest darstellen und
R₃ eine Methylgruppe bedeutet,
das umfaßt:
(a) die Umsetzung einer Verbindung der Formel (18) 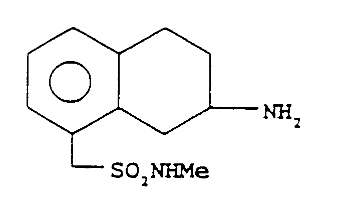 mit einem geeigneten Alkylhalogenid in einem basischen Medium bei leicht erhöhten Temperaturen,
(b) gegebenenfalls Umwandlung der erhaltenen Verbindung (I) in ihre Enantiomere oder pharmazeutisch verträglichen Salze.

12. Verwendung der Verbindungen nach den Ansprüchen 1 bis 9 zur Herstellung von Arzneien, die zur Behandlung von Migräne verwendbar sind.

## Revendications (Revendications pour l'(les) Etat(s) contractant(s) suivant(s): AT, BE, CH, LI, DE, DK, FR, GB, IT, LU, NL, SE)

1. Composé de formule 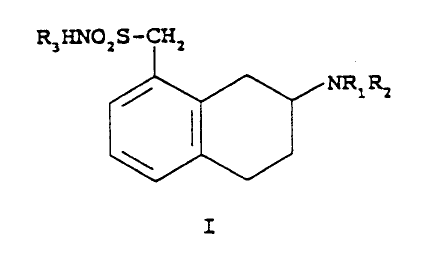 ses énantiomères et leurs mélanges et leurs sels pharmaceutiquement acceptables dans lesquels
R₁ et R₂ représentent chacun un hydrogène ou un alkyle inférieur en C₁₋₇, et
R₃ est un alkyle inférieur en C₁₋₇.

2. Composé selon la revendication 1, dans lequel R₁ et R₂ représentent un C₁₋₇ alkyle.

3. Composé selon la revendication 1, dans lequel R₁ et R₂ représentent H.

4. Composé selon la revendication 1, dans lequel R₁ est H et R₂ est un C₁₋₇ alkyle.

5. Composé selon la revendication 1, dans lequel R₁ et R₂ représentent un n-propyle.

6. Composé selon la revendication 5, dans lequel R₃ est un méthyle.

7. Composé selon la revendication 1, dans lequel R₁, R₂ et R₃ représentent un méthyle.

8. Composé selon la revendication 1, dans lequel R₁ est H et R₂ est un méthyle.

9. Composé de formule I selon la revendication 1 qui est le 2-(N',N'-dipropylamino)-8-méthylaminosulfonylméthyl-1,2,3,4-tétrahydronaphtalène.

10. Procédé de préparation d'un composé de formule 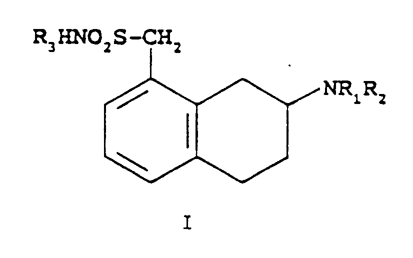 ses énantiomères et leurs mélanges et leurs sels pharmaceutiquement acceptables dans lesquels
R₁ et R₂ représentent chacun un hydrogène ou un alkyle inférieur en C₁₋₇, et
R₃ est un alkyle inférieur en C₁₋₇,
qui comprend
(a) la réaction d' un composé de formule 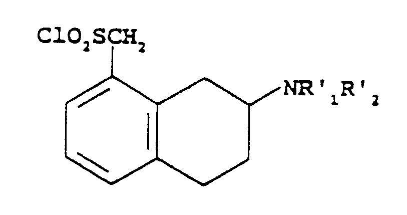 avec une amine de formule NH₂R₃,
dans laquelle R'₁ et R'₂ représentent un C₁₋₇ alkyle ou un groupe protecteur d'amino éliminable, puis l'élimination de tout groupe protecteur éventuel,
(b) la N-alkylation éventuelle du composé de formule 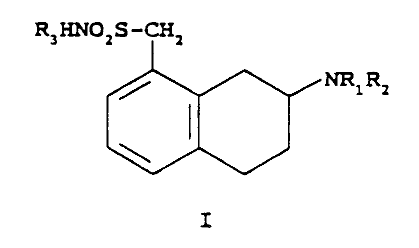 dans laquelle R₁ et R₂ représentent tous les deux un hydrogène
avec un halogénure de C₁₋₇ alkyle,
(c) la transformation éventuelle du composé (I) résultant en ses énantiomères ou ses sels pharmaceutiquement acceptables.

11. Procédé de préparation d'un composé de formule 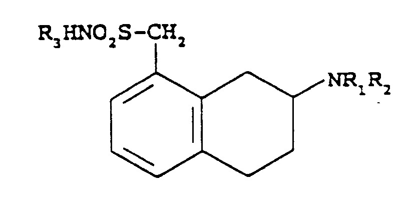 ses énantiomères et leurs mélanges et leurs sels pharmaceutiquement acceptables dans lesquels
R₁ et R₂ représentent chacun un hydrogène ou un alkyle inférieur en C₁₋₇, et
R₃ est un groupe méthyle,
qui comprend :
(a) la réaction d'un composé de formule (18) : 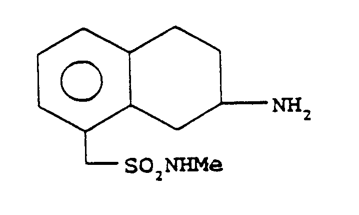 avec un halogénure d'alkyle approprié dans un milieu basique à des températures modérées,
(b) la transformation éventuelle du composé résultant (I) en ses énantiomères ou ses sels pharmaceutiquement acceptables.

12. Utilisation des composés selon les revendications 1 à 9 comme agent pharmaceutiquement actif.

13. Utilisation des composés selon les revendications 1 à 9 pour préparer des médicaments utiles pour le traitement de la migraine.

## Revendications (Revendications pour l'(les) Etat(s) contractant(s) suivant(s): ES)

1. Procédé de préparation d'un composé de formule 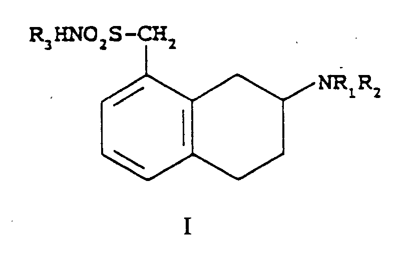 ses énantiomères et leurs mélanges et leurs sels pharmaceutiquement acceptables dans lesquels
R₁ et R₂ représentent chacun un hydrogène ou un alkyle inférieur en C₁₋₇, et
R₃ est un alkyle inférieur en C₁₋₇,
qui comprend
(a) la réaction d'un composé de formule 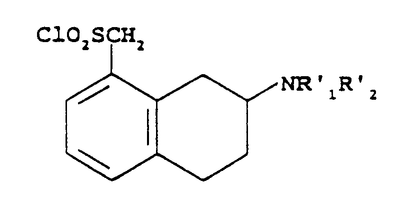 avec une amine de formule NH₂R₃,
dans laquelle R'₁ et R'₂ représentent un alkyle C₁₋₇ ou un groupe protecteur d'amino éliminable, puis l' élimination de tout groupe protecteur éventuel,
(b) la N-alkylation éventuelle du composé de formule 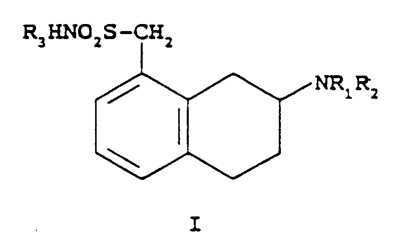 dans laquelle R₁ et R₂ représentent tous les deux un hydrogène
avec un halogénure de C₁₋₇ alkyle,
(c) la transformation éventuelle du composé (I) résultant en ses énantiomères ou ses sels pharmaceutiquement acceptables.

2. Procédé de préparation d'un composé de formule 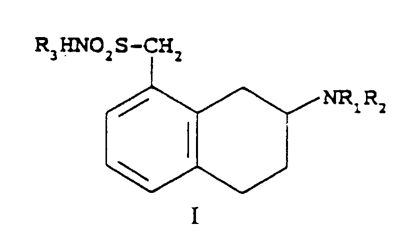 ses énantiomères et leurs mélanges et leurs sels pharmaceutiquement acceptables dans lesquels
R₁ et R₂ représentent chacun un hydrogène ou un alkyle inférieur C₁₋₇, et
R₃ est un groupe méthyle ;
qui comprend :
(a) la réaction d'un composé de formule (18) : 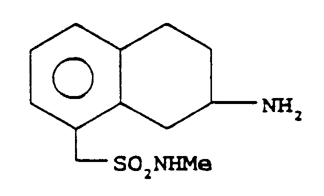 avec un halogénure d'alkyle approprié dans un milieu basique à des températures modérées,
(b) la transformation éventuelle du composé résultant (I) en ses énantiomères ou ses sels pharmaceutiquement acceptables.

3. Procédé selon la revendication 2, dans lequel l'halogénure d'alkyle approprié est un 1-iodopropane et le produit résultant est le 2-(N',N'-dipropylamino)-8-méthylaminosulfonylméthyl-1,2,3,4-tétrahydronaphtalène.

4. Utilisation des composés obtenus par le procédé selon l'une quelconque des revendications 1 à 3 pour préparer des médicaments utiles pour le traitement de la migraine.

## Revendications (Revendications pour l'(les) Etat(s) contractant(s) suivant(s): GR)

1. Composé de formule 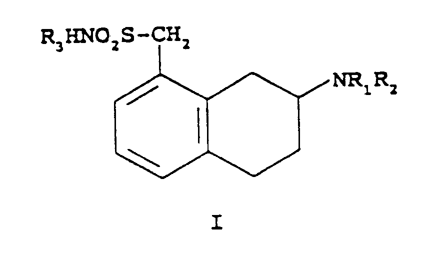 ses énantiomères et leurs mélanges et leurs sels pharmaceutiquement acceptables dans lesquels
R₁ et R₂ représentent chacun un hydrogène ou un alkyle inférieur en C₁₋₇, et
R₃ est un alkyle inférieur en C₁₋₇.

2. Composé selon la revendication 1, dans lequel R₁ et R₂ représentent un C₁₋₇ alkyle.

3. Composé selon la revendication 1, dans lequel R₁ et R₂ représentent H.

4. Composé selon la revendication 1, dans lequel R₁ est H et R₂ est un C₁₋₇ alkyle.

5. Composé selon la revendication 1, dans lequel R₁ et R₂ représentent un n-propyle.

6. Composé selon la revendication 5, dans lequel R₃ est un méthyle.

7. Composé selon la revendication 1, dans lequel R₁, R₂ et R₃ représentent un méthyle.

8. Composé selon la revendication 1, dans lequel R₁ est H et R₂ est un méthyle.

9. Composé de formule I selon la revendication 1, qui est le 2-(N',N'-dipropylamino)-8-méthylaminosulfonylméthyl-1,2,3,4-tétrahydronaphtalène.

10. Procédé de préparation d'un composé de formule 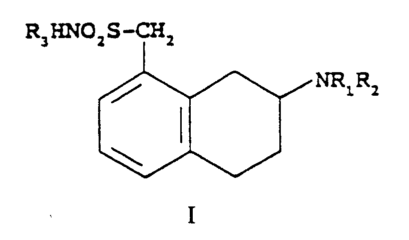 ses énantiomères et leurs mélanges et leurs sels pharmaceutiquement acceptables dans lesquels
R₁ et R₂ représentent chacun un hydrogène ou un alkyle inférieur en C₁₋₇, et
R₃ est un alkyle inférieur en C₁₋₇,
qui comprend
(a) la réaction d'un composé de formule 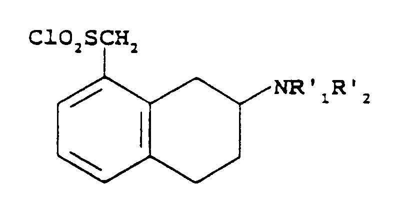 avec une amine de formule NH₂R₃,
dans laquelle R'₁ et R'₂ représentent un C₁₋₇ alkyle ou un groupe protecteur d'amino éliminable, puis l'élimination de tout groupe protecteur éventuel,
(b) la N-alkylation éventuelle du composé de formule 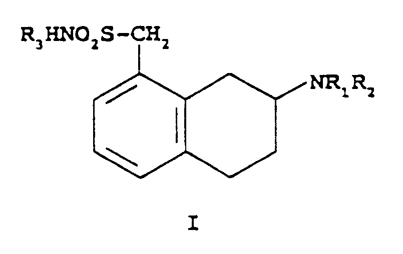 dans laquelle R₁ et R₂ représentent tous les deux un hydrogène
avec un halogénure de C₁₋₇ alkyle,
(c) la transformation éventuelle du composé (I) résultant en ses énantiomères ou ses sels pharmaceutiquement acceptables.

11. Procédé de préparation d'un composé de formule 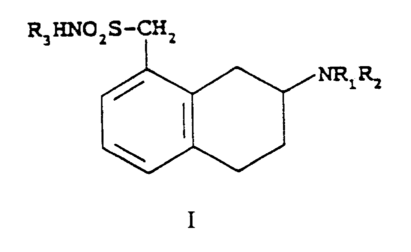 ses énantiomères et leurs mélanges et leurs sels pharmaceutiquement acceptables dans lesquels
R₁ et R₂ représentent chacun un hydrogène ou un alkyle inférieur en C₁₋₇, et
R₃ est un groupe méthyle ;
qui comprend :
(a) la réaction d'un composé de formule (18) : 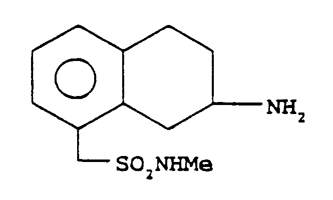 avec un halogénure d'alkyle approprié dans un milieu basique à des températures modérées,
(b) la transformation éventuelle du composé résultant (I) en ses énantiomères ou ses sels pharmaceutiquement acceptables.

12. Utilisation des composés selon les revendications 1 à 9 pour préparer des médicaments utiles pour le traitement de la migraine.
